# EUROPEAN PATENT APPLICATION

(11) **EP 4 838 418 A2**
(43) Date of publication of application: **11.11.2026**
(21) Application number: 26201444.2
(22) Date of filing: 28.08.2025
(51) Int. Cl.: G06N 3/048

(54) **POWER-EFFICIENT METHOD FOR ESTIMATING A USER'S GAZE AND EYE FEATURES AND A HEAD-MOUNTED EYE-TRACKING DEVICE USING THE METHOD**

(30) Priority: 21.11.2024 WO PCT/EP2024/083187
(62) Document number(s) of the earlier application(s) in accordance with Art. 76 EPC: 25766217.1 / 4 771 467
(71) Applicant: Viewpointsystem GmbH, 1010 Wien (AT)
(72) Inventor: PITTERS, Florian, 1080 Wien (AT)
(74) Representative: Schardmüller Gall-Schuhmann Patentanwälte OG

(57) **Abstract**

The present invention relates to a power-efficient eye-tracking method capable of providing a full range of users' eye features using an edge computing device, embeddable in a general head-mounted device, such as AR/VR/XR or smartglasses. The invention is further focused on an eye-tracking unit and/or a head-mounted device embedding the eye-tracking unit using the invented method.

## Description

### Technical Field

The present invention discloses a power-efficient method for estimating the user's gaze and eye features from eye representation inputs and an eye-tracking unit or a head-mounted device embedding the eye-tracking unit using the method.

An eye-tracking unit, commonly known as an eye/gaze-tracking device, within the scope of this document, refers to a specialised electronic system designed to detect, measure, and analyse ocular movements with a certain degree of accuracy. These devices, typically comprising a combination of eye sensors (e.g. imaging cameras) and software algorithms, monitor the position, movement, and gaze direction of the user's eyes. By capturing data on where a user is looking, an eye-tracking device provides insights into attention, interest, and visual engagement, which may be applied across various fields, including assistive technologies, behavioural research, user interface optimisation, and immersive virtual reality systems.

Due to their ability to non-invasively and accurately interpret human visual attention, eye-tracking devices are increasingly integrated into technologies for medical diagnostics, gaming, advertising analytics, and human-computer interaction.

Head-mounted eye-tracking technology refers to a class of wearable systems specifically designed to track eye movements from a device affixed directly to the user's head (so-called head-mounted device). These devices are generally more accurate in dynamic environments, as they move in concert with the user's head, maintaining a consistent viewpoint for tracking ocular activity. Head-mounted eye/gaze trackers offer a range of designs, each leveraging different configurations of sensors, cameras, and optical components to suit varied application requirements.

Wearable display-integrated eye-trackers, which are built into head-mounted displays (HMDs), such as virtual reality (VR) or augmented reality (AR) headsets are known. These systems employ, for instance, small cameras or optical sensors embedded within the display near the lenses, allowing for real-time gaze tracking alongside immersive visual experiences. They are integral in VR and AR applications where the user's gaze must be continuously tracked to enhance interactive elements, improve rendering techniques (such as foveated rendering), or study user behaviour within a virtual environment.

Smart glasses with embedded eye-tracking technology represent a subset of head-mounted devices where eye-tracking components are integrated into eyewear that resembles traditional glasses. These systems are typically lightweight and designed for extended wear, making them ideal for applications requiring unobtrusive, long-duration tracking, such as behavioural research in naturalistic settings or real-world advertising studies. Smart glasses utilise miniaturised cameras or sensors, usually embedded within the frame, to track eye movement with minimal impact on the wearer's mobility.

Head-mounted eye-tracking devices utilise a range of specialised processors to support the demanding requirements of eye data processing, encompassing real-time gaze detection, tracking, and data interpretation. The processors integrated within these devices-namely, central processing units (CPUs), graphics processing units (GPUs), programmable hardware accelerators like digital signal processors (DSPs) or neural processing units (NPUs), and application-specific integrated circuits (ASICs)-each contribute unique computational advantages tailored to the requirements of continuous, high-precision eye tracking.

Central processing units (CPUs) provide foundational control and general-purpose computing within the device, managing system operations and executing control algorithms that enable seamless interaction between various processing units. The CPU often coordinates the input from optical sensors or cameras and ensures that data is routed efficiently for further processing.

For computationally intensive tasks of image analysis and real-time data visualisation associated with eye tracking, many systems incorporate graphics processing units (GPUs). GPUs are highly suited for parallel processing, a requirement in high-resolution image capture and processing, where rapid computation is essential to identify and interpret eye features accurately. This capability is particularly beneficial in applications such as virtual and augmented reality, where immediate and precise gaze mapping enhances user experience and interactivity.

Hardware accelerators, like digital signal processors (DSPs) or neural processing units (NPUs) are utilised in computer devices to accelerate specific functionality such as video encoding, real-time signal-processing or matrix multiplication. While such hardware accelerators are specialised for a specific task, they can typically be used for a wide range of applications that include this task. In scenarios that demand exceptionally high processing speed and minimal delay, particularly in specialised or medical applications, application-specific integrated circuits (ASICs) may be incorporated. ASICs are custom designed to perform eye data processing functions, delivering high accuracy through dedicated circuitry tailored to the exact computational demands of eye tracking.

By integrating CPUs for system control and GPUs for image processing, head-mounted eye-tracking devices achieve the comprehensive processing capabilities required for accurate, real-time gaze tracking across a variety of environments and applications. However, the eye-tracking inference process is more focused on getting accurate real-time measurements of the user's gaze direction, regardless of the power consumption of the eye-tracking pipeline itself.

### Prior Art

The European Patent EP 4 281 829 describes an advanced eye-tracking kit designed specifically for integration with regular eyeglasses. This invention targets the field of wearable technology, specifically enhancing user interaction and experience by monitoring eye movement and gaze direction. The design outlines a modular system that includes an eye-tracking component fitted onto or embedded within the frame of eyeglasses. The system consists of multiple elements, such as sensors, processors, and communication units, optimised for minimal size to ensure comfort and usability for extended wear.

One of the key features highlighted in the patent is the emphasis on non-intrusive user experience, whereby the eye-tracking kit can be seamlessly attached to conventional eyewear without requiring extensive modifications. This adaptability aims to make eye-tracking more accessible and practical for various applications, including augmented reality (AR), virtual reality (VR), and user interface control systems.

However, EP 4 281 829 is focused on mechanical form factor aspects of the eye-tracking kit, not on reducing its power consumption, improving response times, or saving bandwidth for the unit itself.

In general, the typical software pipeline of eye-tracking head-mounted devices begins with the "representation capture" phase, where sensors take eye representation data, which may be in the form of images of the user's eyes. The visual representation may be a conventional camera image, or an output of electrooculography (EOG) sensors, Dynamic Vision Sensors (DVS), or Micro-Electro-Mechanical Systems (MEMS) sensors. These visual representations are then analysed in the "eye feature detection" stage, where the software identifies key features of the eyes, such as the pupil, iris, and any reflections on the cornea. Once these features are detected, the software moves to the "gaze estimation" phase, where it calculates the direction of the user's gaze based on the eye features and their positions in the corresponding representation and the position of the sensors relative to the eyes. Finally, in the "output interpretation" phase, the software interprets the calculated gaze direction to display it to a companion device or to a remotely connected computer device and/or to interact with the computer device itself, such as by moving a cursor or selecting objects on a display.

For instance, in a typical camera-based eye-tracking pipeline, the first stage is image acquisition and preprocessing. This involves capturing images from infrared or visible light cameras and then performing operations such as denoising, normalisation, and sometimes adjusting the frame rate. These tasks require a moderate to high amount of computational resources, particularly when the system is working with high-resolution or high-frame-rate data. However, their power consumption is generally lower than in later stages, unless extremely fast or detailed imaging is used.

The next stage is pupil and feature detection, where the location of the pupil is determined and specific landmarks such as eyelid position, iris texture, or eye corners are identified. This stage is frequently the most computationally demanding, especially when it relies on convolutional neural networks (CNN) or other compute-intensive vision models. Running inference on such models often increases both processing requirements and power consumption, which is particularly difficult on mobile or embedded systems.

After features are extracted, the gaze estimation stage determines the direction of gaze. This can be done using geometric modelling or machine-learning approaches.

The final stages of the process involve output, streaming, and feedback. This includes transmitting gaze data to other systems, storing it, or triggering user interface feedback in real time. From a computational perspective, these steps tend to be less demanding than detection or estimation, but in wireless systems, data transmission can be a significant source of power consumption.

Overall, the pupil and feature detection stage-particularly when implemented with deep learning-is the most resource-intensive part of the pipeline, both in terms of computation and power usage. Image preprocessing can also become demanding if complex filtering or high-resolution streams are involved, and real-time data streaming can be a considerable power drain in wireless contexts.

### Purposes of the Invention

One objective of the present invention, according to a first of its aspects, is obtaining a power-efficient method for estimating user's gaze and eye features for eye/gaze-tracking devices, capable of executing eye/eye descriptor computations with minimal energy consumption, that can be connected to many possible computer application devices, even to very low computational power processing unit as microcontrollers.

A second objective of the present invention is to provide a method used by an eye-tracking device that is able to significantly optimise latency and strike a better balance between accuracy and responsiveness, being able to provide real-time user eye features.

A third objective of the present invention is to provide an eye-tracking method capable of throughput of low-volume time-series asset data, characterising in full the state of the user's eye and the user's eye behaviour.

A fourth objective of the present invention is to provide an eye-tacking method which is able to prevent high temperatures on the processing unit and on the device itself, due to high-demanding computational steps, which can lead to discomfort to the user.

A further objective of the present invention is to achieve a method capable of detecting a complete list of users' eye features as disclosed in the present specification.

The present invention aims also to provide an eye-tracking unit or a head-mounted device embedding the eye-tracking unit performing the method according to the aspects mentioned above.

Another objective of the present invention is to provide a small eye-tracking device that is configured to not interfere with the user´s nose, and/or with the user´s field of view, once connected to the computer application device.

A further objective of the present invention is to provide an eye-tracking unit that is very lightweight, minimising the total weight of the head-mounted device, and embedding the eye-tracking unit.

Another objective of the present invention is to provide a computer-readable memory implementing the method according to the present invention.

### Summary of the Invention

Hereinafter some technical aspects of the present inventions are summarised, which enable some of the most important purposes to be achieved.

According to a first aspect, this invention relates to a method for estimating the user's gaze and other eye features from eye representation inputs provided by at least an eye sensor arranged in an U-shaped nose bridge portion, compatible with the nose of a wearer, of an eye-tracking unit (or gaze-tracking unit) or of a head-mounted device including the eye/gaze-tracking unit, further comprising an edge computation unit electronically connected to at least one eye sensors by an electronic circuitry, said edge computation unit provided with a machine learning hardware accelerator and configured to perform an inference process designed to receive as input user's eye representation data from the eye sensor and to infer as output an array of user's eye features including user's gaze data within an inference time when in use, said method including at least one neural network step implementing a neural network architecture for localizing at least one user's eye feature in the user's eye representation input, wherein the neural network step input and/or neural network step outputs, and/or the neural network step weights are quantized to 8-bit or fewer and/or the neural network step activations are quantized to 16-bit or fewer.

Such a method causes the neural network to require fewer computational resources, less memory, as well as less bandwidth, resulting in less power consumption from the processing unit.

A second aspect of the present invention is to provide a method used by an eye-tracking device, which is able to provide real-time user eye features, wherein the inference time of the prediction of the user's eye feature array is smaller than the acquisition time of the user's eye representation data from the eye sensor.

A third aspect of the present invention is to provide an eye-tracking method capable of throughput of low-volume time-series asset data, characterising in full the state of the user's eye and the user's eye behaviour in a power-efficient way.

A fourth aspect of the present invention is to provide an eye-tracking method requiring reduced space in memory, by using neural network models fitting the size of the SRAM of the edge processing unit, at least with the maximum activation size of the model. Reducing memory access is typically a significant part of the energy budget in neural network inference.

A fifth aspect of the present invention is to provide an eye-tracking method being able to systematically remove parts of the model that contribute little to its predictive performance of the user's eye features, by using a post-training pruning of the neural network architecture.

A sixth aspect of the present invention is to provide an eye-tracking method trained in a much smaller and more efficient model, still capable of retaining much of the original model's performance, by training it via knowledge distillation.

A seventh aspect of the present invention is to provide an eye-tracking method able to identify more efficient neural network structures tailored for specific eye-tracking tasks and eye-tracking edge computing unit device constraints, by using at least semi-automatic neural architecture search (NAS) taking into account latency of the inference process and/or power consumption on the edge computation unit, and/or the SRAM size of the edge computation unit as well as a predetermined accuracy metric.

According to further aspects, this invention relates to an eye-tracking unit and/or a head-mounted device inferring at least one user's eye features by using the eye-tracking method according to what is claimed in the dependent claims of the present specification.

According to further aspects, this invention relates to any computer device and/or computer memory implementing the eye-tracking method according to what is claimed in the dependent claims of the present specification.

### Brief Description of Drawings

The structural and functional features of the present invention and its advantages with respect to the known prior art will become even clearer from the underlying claims, and in particular from an examination of the following description, made with reference to the attached figures which show a preferred but not limited schematic embodiment of the invented method, system, device, in which:
Figure 1 illustrates an eye-tracking unit 1 using the eye-tracking method according to the present invention.
Figure 2 illustrates an eye-racking module 2 embedding the eye-tracking unit 1 using the eye-tracking method according to the present invention.
Figures 3 illustrates an eye-racking module 2 embedding the eye-tracking unit 1 using the eye-tracking method according to the present invention, once connected with a head-mounted device 3.

### Modes for Carrying out the Invention

In general, this disclosure describes a power-efficient eye-tracking method, implementable in any head-mounted eye-tracking unit/head-mounted device, the eye-tracking unit/head-mounted device thereof and any computer device implementing such a method.

The specifications "right" or "left" or "high" or "low" or "front" or "back" relate to the intended manner of wearing eyeglasses and the head-mounted device/eye tracking device by a human being.

It should highlight that for the nose radix a depression is intended at the root of the nose, which defines the nasal root and the origin of the nose from the point of the glabella. The radix extends inferiorly from the nasion to the level of a horizontal line passing through the lateral canthi, and superiorly from the nasion for an equivalent distance.

Furthermore, for nasal bones are two small oblong bones, varying in size and form in different individuals; they are placed side by side at the middle and upper part of the face and by their junction form the bridge of the upper one-third of the nose.

A spectacle frame, as commonly employed in corrective and non-corrective eyewear, generally comprises a pair of eye-rims (also referred to as rims or eyewires), a nose bridge, a pair of endpieces, two temples (or temple arms), and associated hinges and fasteners.

The eye rims are generally constructed as annular or substantially closed structures adapted for securely receiving, retaining, and supporting corrective or protective lenses. Each eye rim features an inner circumferential groove or seat dimensioned for accommodating the edge of a lens. The rims are configured to maintain said lens in a fixed position relative to the frame structure.

Interconnecting the pair of eye-rims is the nose bridge (or bridge), which functions to support the spectacle frame upon the user's nasal structure. The nose bridge may be formed as a unitary member with the eye-rims or attached thereto via integrally moulded or mechanically secured joints. The bridge may incorporate, or be associated with, a pair of nose pads or nose pad arms, each adapted to contact the lateral surface of the user's nose for improved comfort, retention, and weight distribution. The bridge may be of a keyhole type, saddle type, or any other conventional or structurally advantageous configuration.

The endpieces are structurally contiguous with the lateral portions of each eye-rim and serve as mounting regions for the hinges. The hinges provide pivotal engagement between each endpiece and the corresponding temple, permitting the temples to articulate between an open (wearing) configuration and a closed (storage) configuration. The temples are elongated, typically arcuate members dimensioned to extend rearwardly from the frame, over the ears of the wearer, thereby stabilising and retaining the glasses in the operative position.

All the spectacle frame parts mentioned above are reflected in head-mounted eye-tracking devices.

As already anticipated, nowadays, neural network technology is prominently employed in several computationally demanding steps of the tracking pipeline, in head-mounted eye-tracking devices. One critical application is in the localisation of eye features, such as the pupil and corneal reflections for example. These devices often utilise convolutional or transformer-based neural networks to process near-eye camera images, which capture the eye at close proximity under varying lighting and motion conditions. These networks analyse the images to accurately detect and segment the pupil and other eye landmarks essential for precise tracking. This step is inherently computation-intensive due to the high-resolution data and the deep learning model complexity required to handle real-world variability.

Moreover, head-mounted systems often incorporate neural networks for gaze estimation. These networks may combine convolutional layers with recurrent architectures to model both spatial and temporal characteristics of eye movements, delivering robust gaze direction predictions while compensating for inadvertent head movements and device slippage common in wearable setups. The recurrent components add further computational load because they process sequences of frames to capture dynamic eye behaviour over time.

Additionally, some head-mounted eye trackers apply additional neural network modules for calibration correction and distortion compensation, improving gaze accuracy by learning wearer-specific parameters and correcting lens or parallax effects. These neural corrections demand additional processing, increasing the computational and power requirements.

Thus, in currently available head-mounted eye-tracking devices, the most computationally demanding neural network-powered steps include the processing for pupil and feature localisation, deep models for gaze estimation integrating temporal dynamics, and calibration modules to refine accuracy in challenging wearable conditions. These processes collectively contribute to the high computational and power consumption profile characteristic of advanced head-mounted eye trackers.

The solution proposed by the present invention to the above-mentioned technical drawbacks of known head mounted eye-tracking devices is a method for estimating the user's gaze and other user's eye features from eye representation inputs provided by at least an eye sensor arranged in an U-shaped nose bridge portion, compatible with the nose of a wearer, of an eye/gaze-tracking unit 1 or of a head-mounted device 3 including the eye-tracking unit 1, comprising an edge computation unit 10 electronically connected to at least one eye sensors 12 by an electronic circuitry, said edge computation unit 10 provided with a machine learning hardware accelerator and configured to perform an inference process designed to receive as input user's eye representation data from at least one eye sensor 12 and to infer as output an array of user's eye features including user's gaze data within an inference time when in use, said method including at least one neural network step implementing a neural network architecture for localizing the user's eye feature in the user's eye representation input, wherein the neural network step input and/or neural network step outputs, and/or the neural network step weights are quantised to 8-bit or fewer and/or the neural network step activations are quantized to 16-bit or fewer.

Quantisation is a technique that reduces computational power consumption by decreasing the precision of the numerical values used to represent a model's weights and activations. Typically, neural networks use 32-bit floating-point numbers for these computations. Quantisation converts these to lower precision formats, such as 8-bit integers. This reduction in precision means that the neural network model's parameters in the eye-tracking pipeline and calculations require less memory and bandwidth during transmission inside hardware.

Lower-precision integer arithmetic is much less demanding from a computational perspective compared to floating-point arithmetic. In addition, quantized models take up less space in memory, which means data can be accessed and moved more efficiently-a major contributor to overall power savings. Memory access is typically a significant part of the energy budget in neural network inference in eye-tracking inference process, so shrinking the data size with quantisation leads to further energy savings.

Quantisation may also enable better use of specialised low-power hardware as edge computing units, such as machine-learning accelerators and microcontrollers that are optimised for integer math. In fact, a further preferred embodiment of the power-efficient method is configured to cause, during operations, the edge computation unit 10 electronically connected to the eye sensor 12 to use a maximum of 200 mW, preferably a maximum of 100 mW, most preferably a maximum of 50 mW of power, wherein the edge computation unit 10 is a microcontroller.

In a further preferred aspect, the inference time of the user's eye features array is smaller than the acquisition time of the user's eye representation data from the eye sensor 12, allowing the method to provide real-time user's eye features.

The output, being the array of the user's eye features, is provided with respect to the extrinsic parameters (rotations about the axes x, y, z of the original coordinate system of the eye-tracking unit 1) or of the head-mounted device 3 embedding the eye-tracking unit 1.

The neural network step output, being the array of the user's eye features, may include x, y, z-components of the 3D user's gaze directions and according to further possible embodiments, it may include other user's eye features which are selected from the following list: a pupil plane, a 2D pupil centre, a confidence value for the 2D pupil centre, a major radius of the 2D pupil ellipse, a confidence value for the major radius of the 2D pupil ellipse, a minor radius of the 2D pupil ellipse, a confidence value for the minor radius of the 2D pupil ellipse, an orientation of the 2D pupil ellipse, a confidence value for the orientation of the 2D pupil ellipse, a pupil centre ray, a projected pupil centre ray, a 3D extrusion of the projected pupil centre ray, a depth plane, an eyeball centre plane, a 3D eyeball centre, a confidence value for the 3D eyeball centre, a 3D pupil centre, a confidence value for the 3D pupil centre, a distance between the eyeball centre and an eye camera, an eyeball radius, an optical axis, a visual axis, a line of sight, a 3D gaze direction, a monocular 3D gaze direction, a confidence value for the monocular 3D gaze direction, a vergence angle, a binocular 3D gaze point, a confidence value for the binocular 3D gaze point, a binocular 2D gaze point, a confident value for the binocular 2D gaze point, a pupil diameter, a confidence value for the pupil diameter, an apparent pupil diameter, a pupil area, an iris diameter, a confidence value for the iris diameter, a corneal limbus, a major radius of the corneal limbus, a confidence value for the major radius of the corneal limbus, a minor radius of the corneal limbus, a confidence value for the minor radius of the corneal limbus, an orientation of the corneal limbus, a confidence value for the orientation of the corneal limbus, a distance between the corneal limbus centre and the pupil centre, a curvature of the corneal front surface expressed in keratometric dioptres, a distance between the corneal apex and the pupil centre, a distance between the corneal vertex and the pupil centre, an index of refraction of the cornea, an index of refraction of the anterior chamber of the eye, an index of refraction of the posterior chamber of the eye, an index of refraction of the crystalline lens, a distance between the crystalline lens and the 3D eyeball center, a sclera with certain light scattering properties, an eye open/closed state classification, an angular speed of the eyeball, an angular acceleration of the eyeball, eye movements statistics, a classification of the binocular gaze into blink/fixation/pursuit/saccade/vestibulo-ocular reflex/microsaccade events, statistics for the evaluation of the cognitive load of the user, statistics for the evaluation of the attention of the user, statistics for the evaluation of the awareness of the user, user's eye features and eye statistics for user identification and/or authentication.

In a further embodiment, compatible with all possible variants described in the present specification, the method of the present invention may be configured in such a way that at least one neural network model undergoes a post-training pruning of its architecture.

Architecture pruning directly addresses the fundamental constraints of limited computational resources, memory bandwidth, and energy budgets of low-power edge devices. Channel pruning is particularly valuable for such environments because, unlike other pruning techniques, such as fine-grained pruning or pattern-based pruning, channel pruning creates structured sparsity that maps efficiently to the machine learning hardware accelerators of the edge computing unit 1, without requiring specialised sparse computation libraries. By systematically removing entire channels (feature maps) from e.g. convolutional neural networks based on their contribution to model performance, the number of model parameters and the number of computational operations required during inference is reduced. This leads to a lower memory footprint, reduced power consumption and reduced latency, all while preserving acceptable accuracy levels of the eye-tracking method, making it possible to deploy sophisticated deep learning models on the microcontroller of the edge computing unit 10.

Therefore, post-training pruning in eye-tracking neural network steps may reduce computational power consumption by systematically removing parts of the model that contribute little to its predictive performance. During post-training pruning, these less important elements are identified and eliminated, resulting in a smaller, sparser model.

This reduction in model size means that, during inference, the specific neural network requires fewer operations to process input data. With fewer parameters and activations to compute, the number of arithmetic calculations is significantly decreased. Additionally, because the pruned model occupies less memory, there is a reduction in the amount of data that must be loaded into memory (which may be the SRAM of the microcontroller) of the edge computing unit 10, thus lowering power demands also through efficiency in memory usage. The eye-tracking neural network step performance and accuracy are preserved through careful selection of what is pruned, allowing for computational and energy efficiency gains without compromising the output.

In a further embodiment, compatible with all possible variants described in the present specification, the method of the present invention may be configured in such a way that at least one neural network model is trained via knowledge distillation.

Training via knowledge distillation reduces computational power consumption in eye-tracking neural network steps because it results in a much smaller and more efficient model that is capable of retaining much of the original model's performance. The key advantage from an efficiency standpoint is that the distilled eye-tracking neural network step is designed to have fewer layers and parameters than the "teacher" model. Because of this reduction in model size and complexity, the number of required computations (such as matrix multiplications and activations) is significantly decreased. When deployed, the eye-tracking distilled model requires less memory bandwidth, performs fewer arithmetic operations, and, as a result, uses less power for both inference and storage.

In another embodiment, compatible with all possible variants described in the present specification, the method of the present invention may be configured in such a way that at least one neural network models pre-training architecture is obtained via an at least semi-automatic neural architecture search (NAS) using a reward function that parametrizes latency of the inference process and/or a predetermined maximum power consumption of the edge computation unit 10 electronically connected to the eye sensor 12 during the inference process, and/or the SRAM size of the edge computation unit 10 as well as a predetermined accuracy metric. In the context of neural architecture search (NAS), in a preferred embodiment compatible with all the possible variants disclosed in the present specification, the predetermined maximum power consumption of the edge computation unit 10 electronically connected to the eye sensor 12 during the inference process may correspond to a value of 200 mW, preferably a maximum of 100 mW, most preferably a maximum of 50 mW, or even a lower value according to the hardware constraints imposed by the edge computation unit 10 and/or by the edge computing unit 10 electronically connected to the eye sensor 12 and/or by the eye-tracking unit 1.

Using pre-training via at least semi-automatic neural architecture search (NAS) reduces computational power consumption because this approach identifies more efficient neural network structures tailored for specific eye-tracking tasks, i.e. eye feature localisation, eye feature extraction and so on, and edge computing unit 10 features. NAS systematically explores a large space of possible architectures with the help of algorithms that automate and optimise design choices-which include the type and arrangement of layers and operations-so that the final selected model achieves optimal performance using minimal computational resources.

Consequently, automated or semi-automatic NAS leads to architectures that provide significant reductions in computational power consumption compared to manually crafted designs, while sustaining eye-tracking real-time task performance.

In a further embodiment, compatible with all possible variants described in the present specification, the method is configured in such a way that at least the maximum activation size of the model of the neural network step fits the size of the SRAM of the edge computation unit 10. Most preferably, the method is designed in such a way that the model of the neural network step fits the size of the SRAM of the edge computation unit 10.

Regarding eye-tracking glasses in general, it shall be highlighted that they have at least a part of their frame that has the purpose to retain the lenses, which may be prescription lenses or sun lenses or specific filtering/protective lenses. Furthermore, they have at least a portion, the so-called nose bridge, acting as a spacer between the two lenses, able to create the space for the user´s nose.

Today, VR/AR/XR headsets and so-called smart glasses are widely recognised technologies. In some cases, these devices have a very minimal spectacle frame, including a horizontal upper part provided with an element constituting the nose frame, to have the device placed stably and supported by the nose of the user.

VR headsets are designed to fully immerse users in a computer-generated environment, separating them from the physical world and creating a realistic virtual space. Their main components include high-resolution OLED or LCD display panels positioned close to the eyes, providing stereoscopic 3D images by showing slightly different visuals to each eye. The computing power required for VR can be provided by an external computer or, in standalone devices, through onboard processors.

In contrast, AR headsets are designed to overlay digital information onto the real world, providing a mixed-reality experience that enables users to view and interact with virtual objects within their physical environment. These headsets include transparent or semi-transparent display systems, like optical waveguides or reflective displays, that blend digital imagery with the real world by projecting images through micro-projectors onto the lenses. Processing power for AR applications is generally managed within the headset itself, though some devices may require external computing support for more complex tasks.

Furthermore, Extended Reality (XR) headsets represent a broad category that encompasses the full spectrum of immersive technologies, including elements of both VR and AR, and often add enhanced functionalities for a more versatile user experience. XR headsets aim to merge the immersive capabilities of VR with the interactive, real-world integration of AR, allowing users to switch seamlessly between fully virtual environments, augmented overlays, and combinations of both. With powerful onboard processors or wireless connections to external computers, XR headsets handle complex computations needed for fluid transitions between virtual and augmented experiences, making them suitable for a wide array of applications, from professional training and simulation to entertainment and remote collaboration.

So-called smart glasses, on the other hand, provide a more lightweight experience, offering heads-up display (HUD) capabilities for simpler applications like notifications, navigation, and hands-free interaction. Unlike VR/AR and XR headsets, smart glasses do not focus on immersion and often have lower display resolutions, using compact display modules like Micro-LEDs or monochrome OLEDs.

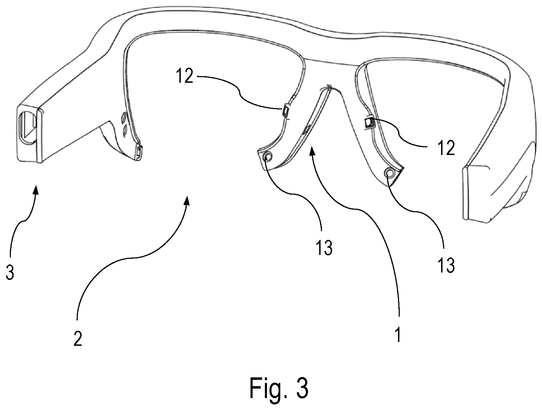

From now on, a general head-mounted device 3 may be for instance any of VR/AR/XR headsets, so-called smart glasses or any electronic device connectable to the eye-tracking module 2 and/or embedding the eye-tracking unit 1 (Fig. 3).

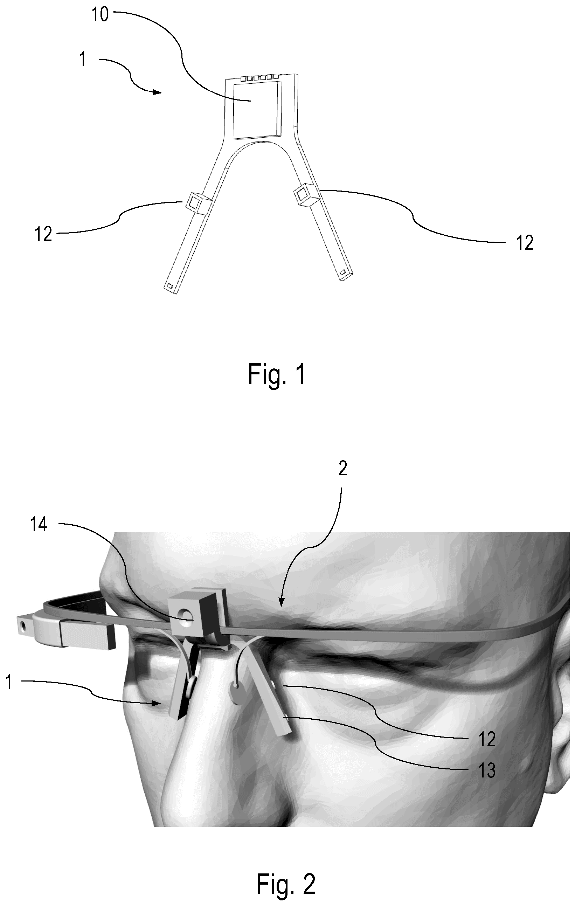

The eye-tracking unit 1 (Fig. 1 or 2), implementing the power-efficient eye-tracking method, according to an embodiment of the present invention, may be a hardware structure which is shaped to be integrated into a wearable U-shaped nose bridge portion compatible with the nose of a wearer when in use, thus being configured to make the head-mounted device 3 easily customizable on different nose shapes when the eye-tracking unit 1 is implemented into said head-mounted device 3. The above-mentioned configuration may be implemented in all the embodiments disclosed in the present specification. The nose bridge is a portion necessary in all eyeglasses, but in some cases, this portion is not clearly detectable, for example, in the ones configured like safety goggles, where a visor replaces the lenses. In some cases, like smart glasses provided with extended technology, the central portion of the upper frame of the smart glasses is provided with an element constituting the nose frame, to have the device placed stably and supported by the nose of the user. Therefore, the eye-tracking unit 1 may be integrated into such a part defined as the nose frame. Another possible mechanical configuration provides for at least the eye sensors 12 of the eye-tracking unit 1 placed in a first PCB nose bridge portion and the edge computing unit in a second PCB portion. In this case, the electronic circuitry of the eye-tracking unit 1 connecting the eye sensors 12 to the edge computing unit 10 is designed accordingly.

The eye-tracking unit 1, is designed according to the edge computing principles, i.e. the computation of the data occurs where or close to where the input data are generated. The eye sensors 12 generate the eye representation data, which are processed directly by the edge computation unit 10 in the eye-tracking unit 1.

The eye-tracking unit 1 (Fig. 1) may be provided with at least one eye sensor 12 facing the user's left eye and/or one eye sensor 12 facing the user's right eye and an edge computation unit 10 provided with a machine learning hardware accelerator configured to receive as input user's eye representation data from the eye sensors 12 and to send as output an array of user's eye features when in use and wherein the edge computation unit 10 may be programmed with a customized bare metal firmware, with a Real-Time Operating System (RTOS) for microcontrollers or a hybrid approach.

With regard to the edge computation unit 10, one preferred solution is that it is a microcontroller programmed with any of the two mentioned approaches, in order to achieve very low power consumption.

Bare-metal systems operate without virtualization layers or host operating systems, enabling application code to execute directly on the underlying hardware with minimal intervening software. This configuration allows applications to interact with and access hardware resources without the overhead of virtualization or the mediation of a general-purpose operating system layer.

In contrast, general-purpose operating systems (GPOS), such as Windows, Linux, and macOS, typically introduce performance overhead due to kernel operations, context switching, and resource management, which can result in jitter and unpredictable delays in response times. By facilitating direct hardware access and eliminating these intermediate layers, bare-metal systems enhance the ability to maintain precise timing and deterministic behaviour, thereby ensuring minimal and predictable latency in system operations.

It may be useful to clarify that a controller is an extremely general term to define a device or system component designed to manage, regulate, or direct the operation of other devices or systems. Controllers execute commands based on inputs they receive, process these commands through programmed logic or algorithms, and produce outputs that adjust or control certain behaviours within a system. They can vary in complexity, from simple analog controllers that respond to electrical signals to complex digital controllers that execute software-based instructions. Controller may be: PLC, Microprocessor, Microcontrollers, FPGA (like for instance ICE40 from Lattice Semiconductors), Motor controller.

Furthermore, it's necessary to distinguish between microprocessor and microcontrollers. According to an article written on 13/6/2024 by Josh Schneider on the IBM website (https://www.ibm.com/think/topics/microcontroller-vs-microprocessor):
*"Microcontroller units (MCUs) and microprocessor units (MPUs) are two kinds of integrated circuits that, while similar in certain ways, are very different in many others. Replacing antiquated multi-component central processing units (CPUs) with separate logic units, these single-chip processors are both extremely valuable in the continued development of computing technology. However, microcontrollers and microprocessors differ significantly in component structure, chip architecture, performance capabilities and application.*

*The key difference between these two units is that microcontrollers combine all the necessary elements of a microcomputer system onto a single piece of hardware. Microcontrollers do not require additional peripherals or complex operating systems to function, while microprocessors do. Both circuits contain CPUs, however, microcontrollers also integrate memory, input*/*output (I*/*O) components and other varied peripherals.*

*Cost-effective and small-in-size, low-power microcontrollers are optimized for all-in-one functionality. As a result, these units are best used for specific applications like automotive infotainment systems and Internet-of-Things (IoT) devices."*

Regarding the hardware configuration, John Schneider explains and distinguishes:

### "Key components of a microcontroller

- *Central processing unit (CPU): Colloquially referred to as the computer's "brain," the CPU is responsible for executing instructions and controlling operations.*
- *Memory: Microcontrollers contain both volatile memory (RAM), which stores temporary data that may be lost if the system loses power, and non-volatile flash memory (ROM) for storing the microcontroller's programming code.*
- *Peripherals: Depending on the intended application, a microcontroller may contain various peripheral components, such as I*/*O interfaces, timers, counters, analog-to-digital converters (ADCs) and communication protocols (UART, SPI, I2C).*
*(...)*

### Key components of a microprocessor

*Modern microprocessors combine millions of small transistors, resistors and diodes assembled on a semiconductor material to create the key components of a CPU.*
- *Arithmetic logic unit (ALU): The main logic unit of the CPU, this component executes logical operations including mathematical calculations and data comparisons.*
- *Control unit (CU): The CU circuit interprets instructions and initiates their execution, directing the basic operations of the processor.*
- *Registers: Small, fast memory storage used by a CPU to temporarily hold data and instructions during computational processes.*
- *Cache memory: Microprocessors and CPUs use cache memory, a high-speed form of memory located close to the CPU, to store frequently accessed data to accelerate performance.*
- *Processor cores: Individual processing units within microprocessors are known as cores. Modern processors frequently incorporate multiple cores (dual-core, quad-core) allowing for parallel processing by enabling the performance of multiple tasks simultaneously.*
- *I*/*O modules: A microprocessor's I*/*O components are critical for managing the flow of data to and from the CPU, including any additional computer peripherals including networking peripherals such as ethernet ports or WiFi units."*

Therefore, a microcontroller (MCU) is a compact, integrated computing device designed for deterministic control of low-power hardware systems. In essence, it is a very specific controller hardware architecture. It operates either without an operating system, known as "bare metal", or with a "Real-Time Operating System (RTOS)", or with a combination of customized bare metal code and Real-Time Operating System RTOS for microcontrollers, providing predictable, time-bound task execution essential for real-time applications and very power efficient, and both of them may be used in the eye-tracking unit 1 of the present invention. Unlike general-purpose processors (like for instance Cortex A architecture as Cortex-A53), microcontrollers do not support general-purpose operating systems (e.g., Windows or Linux) due to their focus on efficient, low-level control and minimal latency requirements as well as their lack of a dedicated Memory Management Unit (MMU).

Microcontrollers are also optimized for low-power operation, with the capability to achieve sub-millisecond wake-up times from sleep modes. This feature allows a microcontroller-based system to enter low-power states between processing intervals or frames, making it suitable for applications with strict power management requirements.

A microcontroller relies on deterministic interrupting handling systems, which ensures that they can respond rapidly to hardware events, typically within a few microseconds. Microcontrollers are designed to prioritize interruptions with minimal latency, allowing them to address high-priority events in real-time. In general-purpose systems, interrupting latency is often less predictable due to the overhead of multitasking operating systems and other background processes.

The interrupt handling, plus a bare metal or RTOS or both allows for a deterministic system, avoiding most of the problems of synchronization.

The left and right eye sensors 12 may be camera-based sensors but may be different kinds of technologies like lasers able to scan user´s eyes to detect position of pupils or micro electromechanical systems like for instance MEMS-based scanning elements.

When the eye sensors 12 are camera-based sensors, they may be monochromatic cameras, dynamic vision sensors (DVS) for example or other cameras. In that case, the eye representations are eye images where at least the user's pupil is included.

When the eye sensors 12 are camera-based sensors the eye-tracking unit 1 may include optics, allowing to control the amount of light that enters the cameras.

When the eye sensors 12 are camera-based sensors the eye-tracking unit 1 may include one or more Infrared LED(s) 13. Infrared (IR) LEDs are components in eye-tracking devices, to provide a source of invisible light that illuminates the eyes without causing distraction, as IR light lies outside the visible spectrum. This illumination helps to capture clear images of the eyes, even in low-light conditions.

The IR light may also enhance contrast for key eye features, such as the pupil and iris. It also creates a bright reflection, known as the "glint," by reflecting off the retina, which helps distinguish the pupil from other parts of the eye.

In order to combine the user eye descriptors as described above, with the field of view of the user, the eye-tracking unit 1, the eye-tracking module 2 and/or the head-mounted device 3 may include a front camera 14. In general, a front camera or said front camera 14 may be arranged such that its field of detection faces the view direction of the user and/or the field of view of the user.

In each embodiment disclosed in the present specification, the output, being an array of user's eye features, may be a text-based file.

In a further embodiment, compatible with all possible variations described in the present specification, the invented method may use the edge computation unit 10 as being configured to be in a sleep state after it has processed the output 20 eye descriptor array and until it receives a wake-up request from a dedicated computer device, e.g. included in the head-mounted device 3.

The present invention furthermore relates to the head-mounted device 3 embedding and/or being connected to the eye-tracking unit 1 and/or the eye-tracking module 2 according to all possible embodiments described in the present specification, implementing the method already described in all its possible variants.

The disclosed invention presents a highly cost-effective and power-efficient eye-tracking unit 1 also called eye/gaze-tracking module, addressing critical challenges in wearable and portable device applications. By leveraging optimised hardware integration, this technology significantly reduces manufacturing costs and operational power requirements without compromising accuracy or reliability. These advancements enable broader accessibility of eye-tracking capabilities across consumer electronics, medical devices, and assistive technologies, promoting widespread adoption while extending device battery life and minimising thermal output. The inventive eye-tracking unit offers a competitive edge by fulfilling industry demands for low-cost, energy-efficient solutions, representing a substantial improvement over existing systems in the field.

A further embodiment of the present invention provides a method for estimating the user's gaze and other user's eye features from eye representation inputs provided by at least an eye sensor arranged in an U-shaped nose bridge portion, compatible with the nose of a wearer, of an eye-tracking unit 1 or of a head-mounted device 3 including the eye-tracking unit 1, said eye-tracking unit 1 or said head-mounted device 3 including the eye-tracking unit 1 further comprising an edge computation unit 10 electronically connected to at least one eye sensors 12 by an electronic circuitry, said edge computation unit 10 provided with a machine learning hardware accelerator and configured to perform an inference process designed to receive as input user's eye representation data from the eye sensor 12 and to infer as output an array of user's eye features including user's gaze data within an inference time when in use, said method including at least one neural network step implementing a neural network architecture for localizing at least one user's eye features in the user's eye representation input, wherein the neural network step input and/or neural network step outputs, and/or the neural network step weights are quantized to 8-bit or fewer and/or the neural network step activations are quantized to 16-bit or fewer.

According to a further embodiment, the inference process outputting an array of user's eye features including user's gaze data is configured to cause, during operations, the edge computation unit 10 electronically connected to the eye sensor 12 to use a maximum of 200 mW, preferably a maximum of 100 mW, most preferably a maximum of 50 mW of power, and wherein the edge computation unit 10 integrates a microcontroller.

According to a further embodiment, the inference time of the user's eye features array is smaller than the acquisition time of the user's eye representation data from the eye sensor 12, allowing the method to provide real-time user's eye features.

According to a further embodiment, at least one or more neural network step outputs are selected from a list consisting of: a pupil plane, a 2D pupil center, a confidence value for the 2D pupil center, a major radius of the 2D pupil ellipse, a confidence value for the major radius of the 2D pupil ellipse, a minor radius of the 2D pupil ellipse, a confidence value for the minor radius of the 2D pupil ellipse, an orientation of the 2D pupil ellipse, a confidence value for the orientation of the 2D pupil ellipse, a pupil center ray, a projected pupil center ray, a 3D extrusion of the projected pupil center ray, a depth plane, an eyeball center plane, a 3D eyeball center, a confidence value for the 3D eyeball center, a 3D pupil center, a confidecnce value for the 3D pupil center, a distance between the eyeball center and an eye camera, an eyeball radius, an optical axis, a visual axis, a line of sight, a 3D gaze direction, a monocular 3D gaze direction, a confidence value for the monocular 3D gaze direction, a vergence angle, a binocular 3D gaze point, a confidence value for the binocular 3D gaze point, a binocular 2D gaze point, a confident value for the binocular 2D gaze point, a pupil diameter, a confidence value for the pupil diameter, an apparent pupil diameter, a pupil area, an iris diameter, a confidence value for the iris diameter, a corneal limbus, a major radius of the corneal limbus, a confidence value for the major radius of the corneal limbus, a minor radius of the corneal limbus, a confidence value for the minor radius of the corneal limbus, an orientation of the corneal limbus, a confidence value for the orientation of the corneal limbus, a distance between the corneal limbus center and the pupil center, a curvature of the corneal front surface expressed in keratometric diopters, a distance between the corneal apex and the pupil center, a distance between the corneal vertex and the pupil center, an index of refraction of the cornea, an index of refraction of the anterior chamber of the eye, an index of refraction of the posterior chamber of the eye, an index of refraction of the crystalline lens, a distance between the crystalline lens and the 3D eyeball center, a sclera with certain light scattering properties, an eye open/closed state classification, an angular speed of the eyeball, an angular accelaration of the eyeball, eye movements statistics, a classification of the binocular gaze into blink/fixation/pursuit/saccade/vestibulo-ocular reflex/microssacade events, statistics for the evaluation of the cognitive load of the user, statistics for the evaluation of the attention of the user, statistics for the evaluation of the awareness of the user, user's eye features and eye statistics for user identification and/or authentication.

According to a further embodiment, at least one neural network step model undergoes a post-training pruning of its architecture, preferably channel pruning of its architecture.

According to a further embodiment, at least one neural network step model is trained via knowledge distillation.

According to a further embodiment, at least one neural network step models pre-training architecture is obtained via an at least semi-automatic neural architecture search NAS using a reward function that parametrizes latency of the inference process and/or a predetermined maximum power consumption of the edge computation unit 10 electronically connected to the eye sensor 12 during the inference process, and/or the SRAM size of the edge computation unit 10 as well as, a predetermined accuracy metric.

According to a further embodiment, at least the maximum activation size of the model architecture of the neural network step fits the size of the SRAM of the edge computation unit 10.

According to a further embodiment, the full model architecture of the neural network step fits the size of the SRAM of the edge computation unit 10.

A further embodiment of the present invention provides a computer-readable storage medium comprising computer-executable instructions which, when executed, configure a processing unit to perform the method described above.

A further embodiment of the present invention provides an eye-tracking unit 1 shaped for being integrated into a wearable U-shaped nose bridge portion compatible with the nose of a wearer, comprising at least an eye sensor 12 and an edge computing unit 10, a computer-readable memory coupled to the edge computing unit 10 said computer-readable memory having stored thereon computer-executable instructions which, when executed, configure the edge processing unit 10 to perform the method described above.

According to a further embodiment, the eye-tracking unit 1 includes an edge computing unit 10 having a machine learning hardware accelerator.

A further embodiment of the present invention provides a head-mounted device 3 comprising the eye-tracking unit 1 described above.

According to a further embodiment, the head-mounted device 3 further comprises eye sensors configured to send data to the edge computing unit 10 to perform the above-described method for estimating the user's gaze from eye representation inputs.

According to a further embodiment, the head-mounted device 3 comprises a front camera 14.

## Claims

1. A method for estimating the user's gaze and other user's eye features from eye representation inputs provided by at least an eye sensor (12) of an eye-tracking unit (1) or of a head-mounted device (3) including the eye-tracking unit (1), said eye-tracking unit (1) or said head-mounted device (3) further comprising an edge computation unit (10) electronically connected to the at least one eye sensor (12) by an electronic circuitry, said edge computation unit (10) configured to perform an inference process designed to receive as input user's eye representation data from the eye sensor (12) and to infer as output an array of user's eye features including user's gaze data within an inference time when in use, said method including at least one neural network step implementing a neural network architecture for localizing at least one user's eye feature in the user's eye representation input, wherein the neural network step input and/or neural network step outputs, and/or neural network step weights are quantized to 8-bit or fewer and/or neural network step activations are quantized to 16-bit or fewer.

2. The method according to claim 1, wherein the eye sensor is arranged in a nose bridge portion of said eye-tracking unit (1) or of said head-mounted device (3).

3. The method according to claim 2, wherein the nose bridge portion is U-shaped, compatible with the nose of a wearer.

4. The method according to any of the preceding claims, wherein the edge computation unit (10) is provided with a machine learning hardware accelerator.

5. The method according to any of the preceding claims wherein the inference process outputting an array of user's eye features including user's gaze data is configured to cause, during operations, the edge computation unit (10) electronically connected to the eye sensor (12) to use a maximum of 200 mW, preferably a maximum of 100 mW, most preferably a maximum of 50 mW of power, and wherein the edge computation unit (10) integrates a microcontroller.

6. The method according to any of the preceding claims wherein the inference time of the user's eye features array is smaller than an acquisition time of the user's eye representation data from the eye sensor (12), allowing the method to provide real-time user's eye features.

7. The method according to any of the preceding claims wherein at least one or more neural network step outputs are selected from a list consisting of: a pupil plane, a 2D pupil center, a confidence value for the 2D pupil center, a major radius of the 2D pupil ellipse, a confidence value for the major radius of the 2D pupil ellipse, a minor radius of the 2D pupil ellipse, a confidence value for the minor radius of the 2D pupil ellipse, an orientation of the 2D pupil ellipse, a confidence value for the orientation of the 2D pupil ellipse, a pupil center ray, a projected pupil center ray, a 3D extrusion of the projected pupil center ray, a depth plane, an eyeball center plane, a 3D eyeball center, a confidence value for the 3D eyeball center, a 3D pupil center, a confidence value for the 3D pupil center, a distance between the eyeball center and an eye camera, an eyeball radius, an optical axis, a visual axis, a line of sight, a 3D gaze direction, a monocular 3D gaze direction, a confidence value for the monocular 3D gaze direction, a vergence angle, a binocular 3D gaze point, a confidence value for the binocular 3D gaze point, a binocular 2D gaze point, a confidence value for the binocular 2D gaze point, a pupil diameter, a confidence value for the pupil diameter, an apparent pupil diameter, a pupil area, an iris diameter, a confidence value for the iris diameter, a corneal limbus, a major radius of the corneal limbus, a confidence value for the major radius of the corneal limbus, a minor radius of the corneal limbus, a confidence value for the minor radius of the corneal limbus, an orientation of the corneal limbus, a confidence value for the orientation of the corneal limbus, a distance between the corneal limbus center and the pupil center, a curvature of the corneal front surface expressed in keratometric diopters, a distance between the corneal apex and the pupil center, a distance between the corneal vertex and the pupil center, an index of refraction of the cornea, an index of refraction of the anterior chamber of the eye, an index of refraction of the posterior chamber of the eye, an index of refraction of the crystalline lens, a distance between the crystalline lens and the 3D eyeball center, a sclera with certain light scattering properties, an eye open/closed state classification, an angular speed of the eyeball, an angular acceleration of the eyeball, eye movements statistics, a classification of the binocular gaze into blink/fixation/pursuit/saccade/vestibulo-ocular reflex/microsaccade events, statistics for the evaluation of the cognitive load of the user, statistics for the evaluation of the attention of the user, statistics for the evaluation of the awareness of the user, user's eye features and eye statistics for user identification and/or authentication.

8. The method according to any of the preceding claims, wherein at least one neural network step model undergoes a post-training pruning of its architecture, preferably channel pruning of its architecture.

9. The method according to any of the preceding claims, wherein at least one neural network step model is trained via knowledge distillation.

10. The method according to any of the preceding claims, wherein at least one neural network step model's pre-training architecture is obtained via an at least semi-automatic neural architecture search (NAS) using a reward function that parametrizes latency of the inference process and/or a predetermined maximum power consumption of the edge computation unit (10) electronically connected to the eye sensor (12) during the inference process, and/or an SRAM size of the edge computation unit (10) as well as a predetermined accuracy metric.

11. The method according to any of the preceding claims, wherein at least a maximum activation size of a model architecture of the neural network step fits a size of an SRAM of the edge computation unit (10).

12. The method according to claim 11, wherein the full model architecture of the neural network step fits the size of the SRAM of the edge computation unit (10).

13. The method according to claim 1, wherein said at least one neural network step is performed by the edge computation unit (10).

14. The method according to any of the preceding claims, wherein the eye sensor (12) is arranged in a first PCB nose bridge portion and the edge computation unit (10) is arranged in a second PCB portion.

15. A computer-readable storage medium comprising computer-executable instructions which, when executed, configure an edge computation unit (10) comprised in an eye-tracking unit (1) or a head-mounted device (3) comprising an eye-tracking unit (1) to perform the method according to any of claims 1 to 14.

16. A computer program comprising instructions which, when executed by an edge computation unit (10) comprised in an eye-tracking unit (1) or a head-mounted device (3) comprising an eye-tracking unit (1), cause the edge computation unit (10) to carry out the method according to any of claims 1 to 14.

17. An eye-tracking unit (1) comprising at least an eye sensor (12) and an edge computing unit (10), said edge computing unit (10) coupled to or comprising a computer-readable memory having stored thereon computer-executable instructions which, when executed, configure the edge computing unit (10) to perform the method according to any of claims 1 to 14.

18. The eye-tracking unit (1) according to claim 17, shaped for being integrated into a wearable, preferably U-shaped, nose bridge portion, compatible with the nose of a wearer.

19. The eye-tracking unit (1) according to claim 17 or 18, wherein the eye sensor (12) is arranged in a first PCB nose bridge portion and the edge computing unit (10) is arranged in a second PCB portion.

20. The eye-tracking unit (1) according to any of claims 17 to 19, wherein the edge computing unit (10) has a machine learning hardware accelerator.

21. A head-mounted device (3) comprising an eye-tracking unit (1) according to any of claims 17 to 20.

22. The head-mounted device (3) according to claim 21, comprising further eye sensors configured to send data to the edge computing unit (10) to perform the method for estimating the user's gaze from eye representation inputs according to any of claims 1 to 14.

23. The head-mounted device (3) according to claim 21 or 22, comprising a front camera (14).
